# EUROPEAN PATENT SPECIFICATION

(11) **EP 1 226 795 B1**
(45) Date of publication and mention of the grant of the patent: **29.03.2006**
(21) Application number: 02001793.5
(22) Date of filing: 25.01.2002
(51) Int. Cl.: A61F 2/01

(54) **Filter device**
Filtervorrichtung
Filtre

(30) Priority: 25.01.2001 US 264175 P; 23.01.2002 US 56588
(43) Date of publication of application: 31.07.2002
(73) Proprietor: ev3 Inc., White Bear Lake, Minnesota 55110-5246 (US)
(72) Inventor: Pavlovic, Jennifer L., Afton, Minnesota 55001 (US)
(74) Representative: Kirschner, Klaus Dieter

(56) References cited:
- WO-A-00/16705
- WO-A-98/51237
- WO-A-99/16382
- WO-A-99/23976
- US-A- 6 001 118
- US-A- 6 110 590

## Description

The present invention relates to devices used in the treatment of stenotic or obstructed vessels or lumens carrying fluid. More specifically, the present invention relates to an improved protection device for the capturing of particulate matter entrained in a vessel while allowing the passage of fluid through the vessel.

In the field of medicine, for example, a substantial health risk exists when deposits of fatty-like substances, referred to as atheroma or plaque, accumulate on the wall of a blood vessel. A stenosis is formed where such deposits form an obstruction restricting or occluding the flow of blood through the blood vessel.

Two different types of procedures during which emboli can become dislodged are commonly used to treat an obstructed region. The first is commonly known in the medical field as balloon angioplasty, wherein the obstruction is deformed by inflating a high pressure balloon to dilate the obstructed region in the vessel prior to inserting a stent. A stent may be deployed in conjunction with the balloon angioplasty. Stent deployment may also result in emboli dislodgement. The second type of treatment is known as an ablation procedure, where all or part of the obstruction is removed from a vessel wall. Ablation procedures, such as thrombectomy and atherectomy procedures, involve mechanically cutting or abrading the stenosis away from the vessel. Other examples of ablation procedures may include the use of lasers, radio frequency (RF) or other common methods which remove an obstruction through the application of heat, pressure, wave frequency, chemical solutions, or commonly known means which do not involve physical contact with the obstruction in order to effect its removal.

During a medical ablation procedure the stenosis is dislodged from the vessel in the form of stenotic debris called emboli. These emboli then become entrained in the blood of the blood vessel and can pose a health risk if the emboli flow to other parts of the vasculature and become lodged therein, creating an occlusion. Blood clots can also form in stasis regions associated with occluded vasculature.

In some of these procedures, there is a risk that a deposit may dislodge causing particulate matter to become entrained in the fluid. Once entrained, the particulate matter may travel downstream and cause a blockage or restrict flow to a smaller vessel elsewhere in the vasculature. This action can cause a stroke or heart attack in the patient. Such risk can be reduced or even eliminated by placing an embolic protection device downstream of the obstruction prior to the deployment of a device for treating the obstruction.

An embolic protection device generally has an elongate shaft or host guidewire, wherein a distal region of the host guidewire has the filter portion of the protection device. Hereinafter, reference to the protection device refers to the filter portion of the protection device. Typically, the filter has an expanded configuration and a collapsed configuration. In the expanded configuration, the protection device expands outwardly from the host guidewire to form a screen or filter having a plurality of pores. The pores act to allow the passage of a fluid, such as blood, through the fluid lumen, while preventing the passage of particulate matter entrained in the fluid. The expanded filter has a diameter at least as large as that of the vessel such that the expanded filter engages the wall of the vessel and traps the entrained material by generally preventing the passage of particulate matter through the pores while still allowing passage of fluid through the pores.

These apparatus typically have a proximal end and a distal end including the protection device. The device acts to prevent the passage of particulate. In one such device, the protection device is advanced across the stenosed region such that the protection device is on the distal side of the stenosis with the guidewire extending from across the other side of the stenosed region. Thus, the protection device is positioned distal to the stenosis with the guidewire extending in a proximal direction.

The protection device may take a variety of shapes. The protection device has a collapsed configuration, wherein the diameter of the protection device is reduced toward the host guidewire. The collapsed configuration has a smaller diameter than the expanded configuration, thus allowing the protection device to be advanced within a vessel of a patient's body.

In general, the protection device must accomplish two things. First, it must prevent the passage of particulate material. Second, it must allow the passage of fluid. The size of particles that are prevented from passage are determined by the pore size of the protection device. The achievable pore sizes and patency of a protection device depend upon the construction of the protection device.

One type of protection device is a protection device comprising a filter having a plurality of woven or braided metal or fabric filaments. The filaments of such devices are relatively large in relation to the size of particulate sought to be captured, thus making small pore sizes difficult to achieve. The construction of such devices having small pores requires a greater number of filaments intersecting and crossing one another. Therefore, these devices constructed in this way are mainly constructed having larger pores so as to filter larger particulate matter and are, therefore, less successful at filtering smaller matter.

Another type of distal protection device employs a film-like material used for construction of the filter, wherein small pores can be cut into the material. The material can then be fitted over a collapsible and expandable frame. Such devices may capture smaller particulate than the intersecting filament device described above, but there is a limit to the smallest pore size that can produced in films using machining or laser drilling techniques. If the film is made thin to more readily permit small pore sizes the film becomes weak. In a further limitation of film devices, the filter material must be folded in the collapsed configuration, leading to difficulty maintaining a smaller diameter, as preferred, in the collapsed configuration.

Both intersecting filament and perforated film devices can have a disadvantage of less open area for the passage of fluid. This results in decreased patency of the filter due to the combination of large non-perforated regions with blood stasis zones distal to these regions, and the comparatively high blood flow rates through the limited number of holes leading to shear activation of thrombus forming blood components. Further, the limited percent open area of these devices renders them susceptible to clogging of the pores with debris, diminishing patency due to mechanical reasons.

WO 99/16382 discloses a perfusion filter catheter which is used to capture potential emboli within the aorta during heart surgery and cardiopulmonary bypass. An expandable embolic filter assembly having fine filter mesh for capturing macroemboli and microemboli is mounted on a catheter shaft having a perfusion lumen with perfusion ports located upstream of the filter. The embolic filter assembly can be actively or passively deployed within the ascending aortic. An optional outer tube covers the embolic filter assembly to prevent premature deployment. Radiopaque markers, sonoreflective markers and/or an aortic transillumination system are provided to monitor the position of the catheter and the deployment state of the embolic filter assembly. The embolic filter assembly is configured to maximize the effective filter surface area when deployed. Embolic filter assembly configurations described include an elongated cone, a frustum of a cone, a trumpet-shape, a modified trumpet-shape, and helically, circumferentially and longitudinally convoluted shapes, as well as configurations having standoff members for centering the filter and holding the filter mesh away from the aortic walls when deployed. Oxygenated blood is perfused through the perfusion lumen and any embolic materials that might be dislodged are captured in the deployed embolic filter assembly. Embodiments are also described that combine the perfusion filter catheter with an aortic occlusion device, which may be a toroidal balloon, an expandable balloon or a selectively deployable external catheter flow control valve. The combined device allows percutaneous transluminal administration of cardiopulmonary bypass and cardioplegic arrest with protection from undesirable embolic events.

Similar problems exist in many other fields, wherein fluid is transferred through a lumen/vessel.

Thus, there remains a need for a protection device that utilizes a small pore size for capturing small particulate yet has a large open area for greater patency in allowing the passage of fluid through the filtering device.

It is an object of the invention to provide a medical device for preventing passage of particulate material entrained in a fluid flowing through a lumen which device is improved with respect to the deficiencies of the prior art discussed above.

For achieving this object, the medical device of the invention comprises the features of claim 1, the sub-claims characterizing advantageous embodiments of the invention.

Preferred embodiments of the device include a collapsible and expandable filter, wherein the filter has a wire frame and a fiber matrix secured to the wire frame. The filter has a shape as determined by the configuration of the wire frame and a pore size, patency, and crossing profile as determined by the fiber matrix secured to the wire frame.

The device may be applied to protection devices for use during a medical procedure in which particulate matter may become entrained in a patient's blood flowing through a blood vessel.

The wire frame includes a plurality of wires crossing one another so as to form a wire frame. The fiber matrix includes a fiber or a plurality of fibers secured to the wire frame. The fiber is applied over the wire frame. The fibers have some elasticity so they move with the frame.

The filter formed from the fiber matrix and attached to the wire frame has a plurality of pores. The pores have a boundary formed from intersecting lengths of fiber or wire or a combination thereof. The wires of the wire frame have a first diameter and the fibers from the fiber matrix have a lesser diameter.

The frame will reinforce the filter such that a filter can be made with fine pore size and the combination will have better strength and finer pore size than by use of either a frame or a fiber matrix alone.

A distal protection device includes a host wire and an expandable, collapsible filter. The filter is preferably secured to the host guidewire at a distal region of the host guidewire. In the expanded configuration, the filter has a periphery expanding outwardly from the host guidewire. In the collapsed configuration the periphery is collapsed toward the host guidewire. The filter in the collapsed configuration has a low-profile diameter, also called a crossing profile, for positioning the distal protection device in a lumen. In the expanded configuration, the filter has a diameter at least as large as that of the lumen diameter. The filter in the expanded configuration prevents the passage of particulate material entrained in a fluid in the lumen while allowing the passage of the fluid.

According to an aspect of the invention, there is provided a medical device for filtering a fluid in a lumen of a patient's body as described in claim 1.

According to a preferred aspect of the invention, the filter is collapsible prior to deployment from a constraining wall.

According to a preferred aspect of the invention, the constraining wall is a catheter.

According to a preferred aspect of the invention, the wire frame is metallic or non-metallic.

According to a preferred aspect of the invention, the fiber matrix and the wire frame together form boundaries defining a multiplicity of pores.

According to a preferred aspect of the invention, the filter is self expanding or includes means for expansion.

According to a preferred aspect of the invention, the fiber is formed by an electrospinning process and is applied in substantially a single strand.

According to a preferred aspect of the invention, the fibers are applied in a flowable state.
According to a preferred aspect of the invention, the fiber matrix is woven in a regular or in a random pattern.

According to a preferred aspect of the invention, the wire frame is braided.

According to a preferred aspect of the invention, the size of each pore is less than 100 microns.

According to a preferred aspect of the invention, a percent open area of the filter is greater than 40%.

According to an aspect of the invention, there is provided a medical device, for use in a lumen of a human body for preventing passage of particulate matter and allowing passage of a fluid, such as blood, comprising a guidewire having an expandable and collapsible filter attached at a distal end, wherein the filter has a collapsed configuration wherein the filter is able to be advanced within the lumen; and an expanded configuration wherein the filter is expanded outward from the guidewire to engage a wall of the lumen, the filter having fibers defining a plurality of pores, the pores allowing passage of blood and preventing passage of particulate matter therethrough, the filter comprising a metal frame having a plurality of metal wires on which the fiber is spun to form a fiber matrix.

According to an aspect of the invention, there is provided a medical device for filtering fluid passing through a lumen in a patients body, comprising a flexible frame including a plurality of wires intersecting to define a perimeter of an open space; and a matrix including a fiber extending across the open space to define a multiciplity of pores.

According to a preferred aspect of the invention, the device further comprises a retainer to hold the flexible frame in a collapsed configuration for insertion into the lumen to be deployed therein.

According to a preferred aspect of the invention, the flexible frame is carried by a guidewire, and wherein the retainer comprises a catheter having a lumen within which the flexible frame, in its collapsed configuration, is received prior to deployment.

According to a preferred aspect of the invention, the plurality of wires intersect to define perimeters of a multiplicity of open spaces, each open space having a matrix including a multiplicity of fibers extending thereacross to define a multiplicity of pores.
According to a preferred aspect of the invention, each of the pores is generally parallelogram shaped or square shaped or diamond shaped or irregularly shaped.

According to a preferred aspect of the invention, the flexible frame, when deployed, is generally windsock shaped.

According to a preferred aspect of the invention, the flexible frame is made of Nitinol.

According to a preferred aspect of the invention, each of the wires has a diameter of between 0,0038 cm (0.0015 inches) and 0,012 cm (0.005 inches).

According to a preferred aspect of the invention, the fiber is electrospun directly onto the flexible frame.

Embodiments of the invention are now described with reference to the attached drawings in which:
FIG. 1 is a medical device embodiment of the present invention, wherein a distal protection device with a host wire is deployed distal to a stenosed region for the capture of particulate, wherein a working device is positioned over the host wire for treatment of the stenosed region;

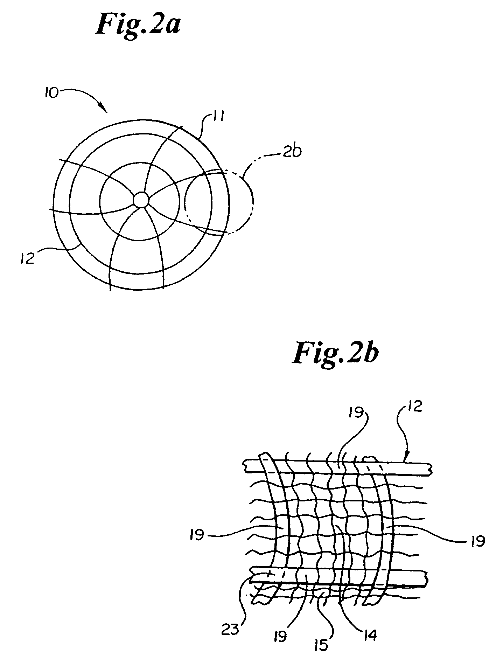

FIG. 2a illustrates a configuration of a wire frame constructed for use in an embodiment of the present invention;
FIG. 2b illustrates a section of a wire frame having a fiber matrix secured to the frame constructed for use in an embodiment of the present invention;
FIG. 3 illustrates an embodiment of the present invention in a collapsed configuration;
FIG. 4 illustrates an embodiment of the present invention in an expanded configuration for capture of particulate matter;
FIG. 5a illustrates a fiber matrix having a random weave fiber matrix constructed for use in an embodiment of the present invention;
FIG. 5b illustrates a fiber matrix having an angled weave constructed for use in an embodiment of the present invention;
FIG. 5c illustrates a fiber matrix having an aligned weave constructed for use in an embodiment of the present invention;
FIG. 5d illustrates a non-woven fiber matrix;
FIG. 6 illustrates a filter of a distal protection device having alternative shapes for use with an embodiment of the present invention; and
FIG. 7a-7c, 8 and 9 each illustrate an alternate embodiment.

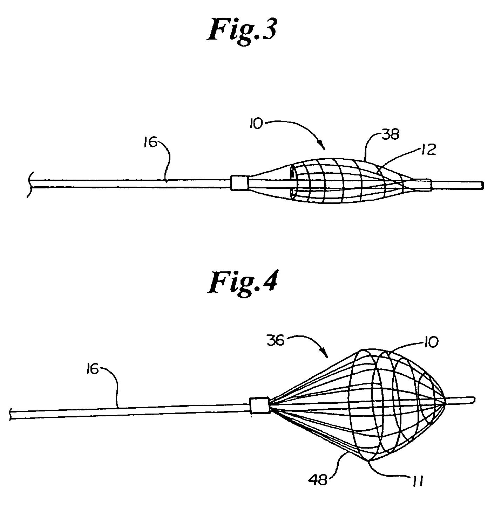

The present invention embodies an expandable filter 10 for use in a distal protection device 36. The distal protection device 36 comprises the filter 10 attached to a guidewire 16. The protection device 36 has an expanded configuration (as seen in FIG. 4) and a collapsed configuration (as seen in FIG. 3). In the expanded configuration the filter 10 has a periphery 11 extending outwardly from guidewire 16. In the collapsed configuration of FIG. 3, the periphery 11 of the filter 10 collapses towards the guidewire 16. The filter 10 has a wire frame 12 over which is overlain a fiber matrix 14. The filter 10 thereby defines a plurality of pores 15. The pores 15 have a boundary formed by one or more fibers, wires, or a combination thereof.

In use, the filter 10 is positioned in a lumen 22 by advancing the distal protection device 36 through the lumen 22 in the collapsed configuration shown in FIG. 3. Once positioned, the distal protection device is deployed into the expanded configuration as shown in FIG. 4.

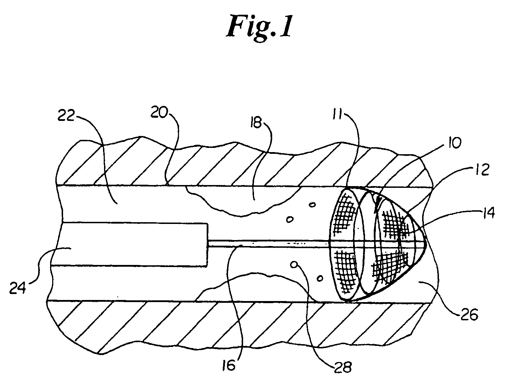

FIG. 1 illustrates an embodiment of the current invention in the lumen 22 of a patient's body, such as a blood vessel 22. The filter 10 is deployed to attain the expanded configuration in a position distal to a stenosis 18. The blood vessel 22 has a diameter, wherein the periphery 11 of the filter 10 in the expanded configuration is at least as large as the diameter of the blood vessel, so as to prevent emboli 28 from bypassing the filter 10. A working device 24 having a central lumen is positioned over the guidewire 16 for treatment of the stenosis 18. During treatment of the stenosis 18 the working device 24 may cause particulate matter 28 such as emboli to become entrained in a fluid, such as blood, flowing in the blood vessel 22. The filter 10 prevents passage of a proportion of particulate matter 28, while allowing the flow of the fluid through the lumen 22. Particulate matter 28 having a given size is prevented from passing through the pores 15 of the filter 10 where the pores 15 have a size less than that of the particulate matter 28.

Once the stenosis 18 has been treated, the distal protection device 36 returns to the collapsed configuration, wherein the particulate matter 28 is captured within the filter 10. The working device 24 and distal protection device 36 are then removed from the lumen 22 with the particulate matter 28 captured by the filter 10 also removed from the lumen 22 therewith. Alternatively, the particulate matter can be removed in whole or in part from the filter by means of aspiration, or by transference of the particulate matter to a recovery catheter, and the device can then be collapsed and withdrawn.

Alternatively, a working device 24, especially adapted for crossing a stenosis, is used to deliver the filter downstream of the stenosis. Such working device may be a catheter such as is typically used for balloon angioplasty, stent delivery, or stent deployment, or a single or multi-lumen catheter compatible with the filter.

The filter 10 comprises a fiber matrix 14 overlying a wire frame 12. The fiber matrix 14 conforms to the shape of spaces defined by the wire frame 12 allowing the filter to have numerous shapes and configurations. The wire frame 12 comprises a plurality of individual wires 19. Wire frame 12 has a shape determined by the relative orientation of the wires 19 of the frame 12. Each individual wire 19 can have a helical-type configuration, wherein a first wire 19 will have a rotation in one direction and a second wire 19 will have an opposite rotation.

The overall shape of the wire frame 12 depends on how each of the wires 19 intersect and cross one another and also upon the use of wire frame shape setting. This will depend on the pitch and pick of the wires 19 where the pitch is the angle defined between the turns of the wire and the axis of the braid and the pick is the number of turns per unit length. The pitch and pick may vary along the length of a given wire 19, thus allowing the wire frame 12 to have a plurality of shapes and configurations. The wire frame 12 defines a plurality of open spaces between adjacent wires 19. The open spaces have a boundary formed from one or more wires. Pores 15 may be shaped as a square, a diamond, or a paralellogram, or other shapes as determined by the pitch and pick of the wires 19, including irregular shapes for example in the case of randomly dispersed fibers. The size of the pore is also determined by the make-up of the wire frame 12 such that a pore having a boundary, the sides of which may be of a predetermined length, may be adjusted with the pitch and/or pick of the wire 19. The same adjustment of the size of a boundary of a pore 15 may also be made for alternative shapes of the wire frame 12.

The wire frame 12 itself is not limited to any particular shape. For instance, FIG. 1 illustrates the wire frame 12 in a basket shape, but the wire frame 12 may take a shape of a windsock, a bell, several shapes in series, and so on. The wire frame 12 is thus not limited to the shapes illustrated in the figures herein provided.

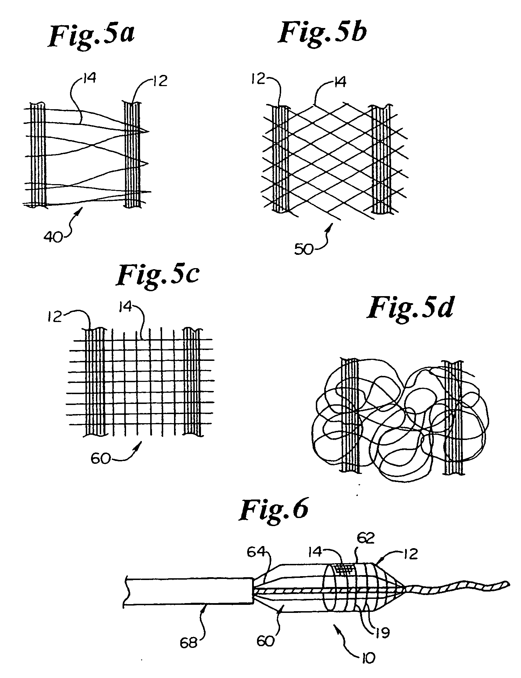

The wire frame 12 has two configurations, an expanded configuration and a collapsed configuration regardless of its shape. In the expanded configuration, the wires forming the wire frame 12 expand, generally outward from the guidewire 16, forming a periphery having a predetermined shape. In the collapsed configuration, the periphery of the wire frame 12 collapses towards the guidewire 16 allowing the wire frame 12 to advance through a lumen 22. In the collapsed configuration, the wire frame can be advanced within the lumen 22 to a predetermined position within the lumen. Once positioned within the lumen, the wire frame is expanded, either manually or self-expanded, to its expanded configuration, wherein the periphery of wire frame 12 is at least as large as the wall defining the lumen 22, such as that of the wall of a blood vessel 22. The wire frame 12 is able to alternate between the expanded configuration and collapsed configuration by use of means for expansion. A plurality of tethers, secured to the periphery of the wire frame 12, can allow the wire frame 12, in the expanded configuration 40, to be drawn into a collapsed configuration, and then returned to substantially the same expanded configuration. Struts (shown in FIG. 6) can serve to expand and contract the wire frame 12.

Another means for expansion comprises a guidewire 16 having an inner core wire secured to a first end of the wire frame 12 while an outer wire is secured to a second end of the wire frame 12. As the two ends are moved away from one and other, the periphery collapses toward the guidewire 16, and as the two ends are moved toward one and other the periphery expands outward from the guidewire 16. Alternatively the outer wire may be a tube that is coaxial around the outside of the inner core wire.

The means for expansion may be any means by which a first end of the wire frame 12 may be moved away from a second end of wire frame 12 so as to cause the periphery to collapse toward the guidewire 16, and as the ends are moved toward one another the periphery of the wire frame 12 expands outward from the guidewire 16.

The wire frame 12 comprises a plurality of wires 19 that may be of any material sufficient to maintain its shape. For example, metals or polymers are two such suitable materials. Examples of suitable polymers include nylons, polyester, PEEK, polyimide, liquid crystal polymers, Teflon, Tefzel, polyurethanes, shape memory polymers, and the like. Example of suitable metals are elgiloy, MP35N, spring steel, stainless steel, titanium and the like. In a preferred embodiment of the present invention, wires 19 are comprised of a shape memory metal alloy. One such shape memory alloy is a nickel titanium alloy, NiTi, commercially known as Nitinol. A shape memory alloy has a characteristic that once it has been formed to a predetermined shape it can be deformed by a force and will return substantially to the original shape upon removal of the deforming force. Nitinol wires used for a frame 12 preferably have diameters on the magnitude of 0.0015@ to 0.005@. In a preferred embodiment, any number of wires 19 may be used to form the frame 12. Considerations on determining the number of wires 19 used may depend on the shape of the frame 12 and/or the necessary dimensions of the periphery of the frame 12 in the expanded state, and/or other considerations, such as pore size, and the like. The number of wires 19 used in the frame 12 will also depend on the characteristics of the fiber matrix 14 secured to frame 12, and are discussed below.

A fiber matrix 14 is secured to wire frame 12, wherein fiber matrix 14 assumes substantially the shape of wire frame 12. Fiber matrix 14 has a plurality of pores 15, preventing passage of particulate matter 28 at least as large as or larger than fiber matrix pore size. The fiber matrix may be on a distal side of the frame, the proximal side, interwoven therethrough, or any combination of the above.

A preferred embodiment of the fiber matrix 14, comprises a fiber or plurality of fibers having a diameter of about 10 microns and a pore size of about 100 microns. The fibers, thus, have a diameter less than that of the wires 19 of wire frame 12. The smaller diameter of the fibers allows the filter 10 to have a smaller pore size. Further, the periphery of such a filter 10 in the collapsed configuration is substantially less than that of a wire frame 12 with an equivalent pore size. The smaller diameter of the fibers allow for a greater open area for the passage of fluid through the filter 10.

A standard formula is used to calculate the percent open area of a given design. The percent open area is calculated by dividing the total pore 15 area by the total filter 10 area (including the pore area) for a representative average portion of the filter 10. A prior art wire frame with a 100 micron pore size and without an electrospun matrix will have a substantially less open area than the filter 10 having the fiber matrix 14 for the same pore size. For a 100 micron pore size a prior art wire frame will have a percent open area of less than 40%, whereas the filter 10 with fiber matrix 14 will have a percent open area of greater than 80%.

A wire frame in the preferred embodiments will have a larger open space than the fiber matrix pore size. The wire frame percent open area in the preferred embodiments may be larger or smaller than the fiber matrix percent open area depending size and spacing of wires utilized.

The fiber matrix 14 can be formed from a single fiber or a plurality of fibers. Fiber matrix 14 may be secured to wire frame 12 by an electrospinning process, one such process is discussed below.

FIGS. 5a, 5b, 5c, and 5d illustrate fiber matrix 14 electrospun onto wire frame 12 in a random weave 40 (FIG. 5a), aligned weave 60 (FIG. 5c), angled weave 50 (FIG. 5b), non-woven 70 (FIG. 5d), or other suitable patterns. The fiber matrix may be on the distal side of frame, the proximal side, interwoven therethrough, or any combination of the above. Different weaves or non-wovens 40, 50, 60, and 70 can form different pore shapes and sizes. The fiber matrix 14 maintains attachment to, and substantially conforms to the shape of, the wire frame 12 during use and must have sufficient strength to prevent passage of particulate 28.

Any material that forms a fiber with the desired fiber matrix characteristics may be used in the current invention. The materials can be polyurethane, nylon, PEBAX, silicone, or any other flexible polymer suitable for electrospinning. One particularly appropriate material is polylactic acid, hereinafter referred to as PLA. PLA is a biodegradeable substance, however, the fiber matrix 14 need not be comprised of biodegradeable fibers, nor is PLA a limiting material. The fiber matrix 14 disclosed herein is made by an electrospinning process. A suitable electrospinning process for fabricating the present invention is disclosed in *Preliminary Design Considerations and Feasibility of a Biomimicking Tissue-Engineered Vascular Graft*, Stitzel and Bowlin, BED-Vol. 48, 2000 Advances in Bioengineering ASME 2000. One aspect of the present invention involves electrospinning of the fiber directly onto the wire frame 12. The electrospinning process involves a voltage source running to a ground, wherein the fiber is electrospun onto wire frame 12, attached to means for electrospinning spinning. The means for electrospinning causes the wire frame 12 to rotate such that fiber is disposed about the surface of the wire frame 12. The fiber characteristics are affected by the electrospinning process, and, consequently, various parameters must be optimized for electrospinning the fiber.

The function of the fiber matrix 14 is to capture or prevent passage of particulate matter 28. This function is accomplished by attaching the fiber matrix 14 to the metal frame 12 by electrospinning the fiber 14 onto the frame 12. The fiber matrix 14 comprises a single fiber electrospun about metal frame 12.

The fiber matrix 14 must have sufficient strength to capture particulate matter 28 without the fiber matrix 14 being damaged, torn, or broken. The fiber matrix 14, should be constructed such that once attached to wire frame 12, the matrix 14 substantially adopts the shape of the frame 12. The frame 12 may take on one of any of a number of predetermined shapes, and the fiber matrix 14 will assume substantially the shape as the frame 12. The wire frame 12 has an expanded configuration and a collapsed configuration, wherein the fiber assumes substantially the same configuration as the wire frame 12 and is able to transition between the two configurations.

The filter 10 comprises the wire frame 12 and fiber matrix 14, and may assume an expanded configuration or collapsed configuration. The collapsed state of the filter 10 has a low profile (a small diameter) for allowing the filter 10 to more easily be positioned in the lumen 22. The filter 10 has a plurality of pores. The pores have a boundary formed from one or more fibers, wires, or a combination thereof. In the expanded configuration, the filter 10 prevents particulate material 28 having a size larger than the pores 15 from passing distal to filter 10. The filter 10 maintains fluid patency by allowing fluid, such as blood, to pass through filter 10. In one embodiment, the filter 10, or components thereof, may have an antithrombogenic coating so as to prevent an occlusion of the lumen 22. In another embodiment, the filter 10, or components thereof, may have a thrombogenic coating so as to completely occlude the lumen 22 and prevent passage of both particulate matter 28 and fluid.

FIG. 6 illustrates one embodiment of a filter 10 of the present invention comprising a wire frame 12 having a plurality of wires 19 with a diameter of about 0,0025 cm (0.001 inches) to 0,012 cm (0.005 inches). The wire frame 12 has a basket shape and a fiber matrix 14 that is secured to wire frame 12. The fiber matrix 14 is substantially in the shape of the interior of the wire frame 12. The fiber matrix 14 comprises a single fiber or a plurality of fibers preferably having a diameter of about 8 to 10 microns. The fiber matrix 14 is preferably secured to wire frame 12 by an electrospinning process. The filter 10 preferably has a plurality of pores 15 having a size of about 100 microns and a percent open area of about 80%. The filter 10 is secured to a guidewire 16, wherein the filter 10 is centered over guidewire 16 such that the periphery of the filter 10 expands outward from the guidewire 16. The filter 10 and guidewire 16 form a distal protection device 36. The distal protection device 36 has a collapsed configuration, wherein distal protection device 36 is advanced within the lumen 22 to a position distal to a stenosis 18. The distal protection device 36 is then put in an expanded configuration, wherein the periphery of the filter 10 extends outward from the guidewire 36 such that periphery is at least as large as lumen 22 wall.

For medical device applications, the distal protection device 36 may have a working device 24 (as seen in FIG. 1) positioned over guidewire 16 that may be used for treating the stenosis 18. The working device 24 treats the stenosis 18 causing particulate matter 28 to become entrained in blood of blood vessel 22. At least a portion of particulate matter 28 is prevented from flowing distal to distal protection device 36, wherein, after treatment of stenosis 18, distal protection device 36 is returned to a collapsed configuration. Particulate matter 28 that is captured by distal protection device 36 is then removed from blood vessel 22 by removal of distal protection device 36.

Working devices 24 such as an atherectomy or thrombectomy ablation device are commonly known to those skilled in the art. Such working devices 24 are able to receive a guidewire 16 into a central lumen of the working device 24 for positioning in a blood vessel 22 and are used as a means for treatment of a stenosis 18. Various technologies may be employed by a working device 24 as a means for treatment of a stenosis 18. For example, rotating cutting surfaces, use of a catheter, pressurized fluids, and various other means currently known in the art may be utilized. One such working device 24 is described in Drasler, et al U.S. Pat. No. 6,129,697 issued October 10, 2000, and assigned to Possis Medical, Inc..

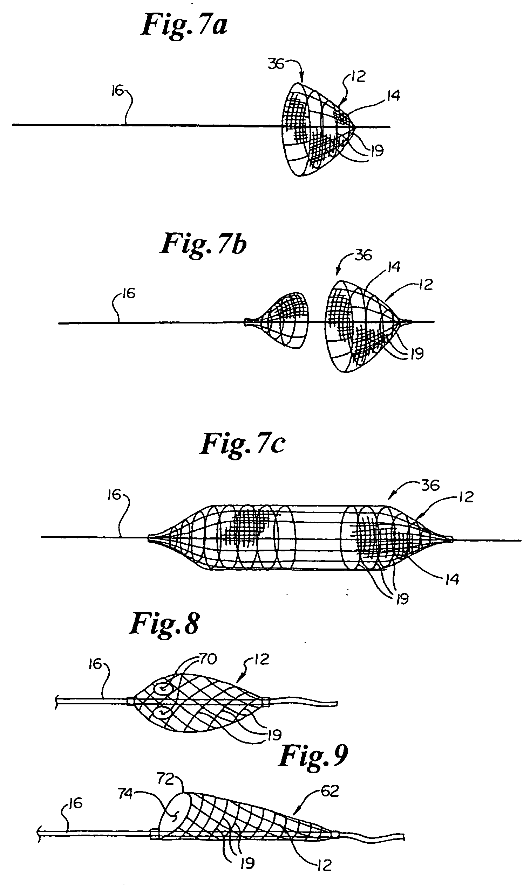

FIG. 7 illustrates an embodiment of filter 10 having a non-woven wire frame 12 expanded by struts 64. Wires 19 of the frame 12 extend outwardly with respect to the guidewire 16, forming filter 10 having an open end 60. The fiber matrix 14 is attached to the wire frame 12 to form a basket 62 with an open end 60. Struts 64 extend from the open end 60 of the basket 62 towards a catheter 68. The catheter 68 can be advanced over the struts 64 so as to collapse the basket 62, or retracted to deploy the struts 64 so as to expand the basket 62.

FIG. 8 illustrates yet another embodiment utilizing the present invention. FIG. 8 illustrates the fiber matrix 14 attached to the wire frame 12 so as to define perimeters about a plurality of openings 70. The openings 70 in FIG. 8 are positioned radially outwardly from the guidewire 16 such that the guidewire 16 does not extend through any of the openings 70.

FIG. 9 illustrates yet another embodiment of the present invention. The fiber matrix 14 is attached to a wire frame 12 so as to define, along with a flexible loop 72, a basket 62 having an open end 74. The basket 62 is positioned non-concentrically about the guidewire 16. The basket 62 is able to receive particulate matter 28 through the open end 74 of the basket 62 and concurrently permit blood flow.

It will be understood that this disclosure, in many respects, is only illustrative. Changes may be made in details, particularly in matters of shape, size, material, and arrangement of part within the scope of the claims. Accordingly, the scope of the invention is as defined in the language of the appended claims.

## Claims

1. A medical device (36) for preventing passage of particulate material (28) entrained in a fluid flowing through a lumen (22) comprising
a collapsible and expandable filter (10), wherein the filter (10) has a wire frame (12), comprising a plurality of wires (19) oriented to define a perimeter, and
a fiber matrix (14), including a multiciplity of fibers extending across an open space defined by the perimeter to define a multiciplity of pores (15), secured to the wire frame (12), **characterized by** that
the fiber matrix (14) comprises a fiber electrospun directly onto the wire frame (12).

2. The device (36) of claim 1, wherein the filter (10) has pores (15) of about 100 microns and a percent open area of about 80 %.

3. The device (36) of claim 1, wherein the wires (19) have a first diameter and the fiber of the fiber matrix (14) has a lesser diameter.

4. The device (36) of claim 1, wherein the wires (19) are selected from metal or polymers.

5. The device (36) of claim 4, wherein the polymers are selected from nylons, Teflon, Tefzel, polyurethanes, shape memory polymers.

6. The device (36) of claim 4, wherein the metals are selected from elgiloy, MP35N, spring steel, stainless steel, titanium, a shape memory metal alloy.

7. The device (36) of claim 1, wherein the fiber of the fiber matrix (14) has a diameter of about 8 to 10 microns and the fiber matrix (14) has a pore (15) size of about 100 microns.

8. The device (36) of claim 1, wherein the fiber of the fiber matrix (14) is selected from polyurethane, nylon, PEBAX, silicone, a flexible polymer suitable for electrospinning, polylactic acid.

9. The device (36) of claim 1, wherein the wires (19) have a diameter of about 0,0025 cm (0.001 inches) to 0,012 cm (0.005 inches).

10. The device (36) of claim 1, further comprising a guidewire (16) having the filter (10) attached at a distal end of the guidewire (16).

11. The device (36) of claim 10, wherein the filter (10) has a collapsed configuration and an expanded configuration (40), wherein:
the filter (10) in the collapsed configuration is collapsed toward the guidewire (16); and
the filter (10) in the expanded configuration (40) is expanded outward from the guidewire (16).

12. The device (36) of claim 1, wherein the fiber matrix (14) is woven in a regular pattern.

13. The device (36) of claim 1, wherein the fiber matrix (14) is woven in a random pattern.

14. The device (36) of claim 1, wherein the fiber matrix (14) is on a distal side of the frame, the proximal side, interwoven therethrough, or any combination of the above.

15. A method for making a medical device (36) according to claim 1 for filtering particulate (28) from a fluid in a lumen (22) of a patient's body, by forming a filter (10) with a wire frame (12) and a fiber matrix (14), **characterized by**
directly electrospinning a fiber onto the wire frame (12).

16. The method of claim 15, wherein the fibers are applied in a flowable state.

17. The method of claim 15, wherein the fiber matrix (14) is woven in a regular or in a random pattern.

## Patentansprüche

1. Medizinische Filtervorrichtung (36) zum Verhindern des Durchtritts von teilchenförmigem Material (28), das in einem Strömungsmittel aufgenommen ist, das durch ein Lumen (22) fließt, umfassend:
einen zusammenfaltbaren und aufspannbaren Filter (10), wobei der Filter (10) einen Drahtrahmen (12), der eine Vielzahl Drähte (19) aufweist, die orientiert sind, um einen Umfang zu definieren, und
eine Filtermatrix (14) hat, die eine Vielzahl Fasern umfasst, die sich über einen offenen, von dem Umfang definierten Raum erstrecken, um eine Vielzahl Poren (15) zu bilden, und die an dem Drahtrahmen (12) befestigt sind, **dadurch gekennzeichnet, dass**
die Fasermatrix (14) eine Faser aufweist, die direkt auf den Drahtrahmen (12) auf elektrischem Wege aufgesponnen ist.

2. Vorrichtung (36) nach Anspruch 1, worin der Filter (10) Poren (15) von etwa 100 Mikron und eine prozentuale Öffnungsfläche von etwa 80% hat.

3. Vorrichtung (36) nach Anspruch 1, worin die Drähte (19) einen ersten Durchmesser und die Faser der Fasermatrix (14) einen geringeren Durchmesser hat.

4. Vorrichtung (36) nach Anspruch 1, worin die Drähte (19) ausgewählt sind aus Metall oder Polymeren.

5. Vorrichtung (36) nach Anspruch 4, worin die Polymere ausgewählt sind aus Nylon-, Teflon-, Polyurethan-, Formspeicher-Polymer-Materialien.

6. Vorrichtung (36) nach Anspruch 4, worin die Metalle ausgewählt sind aus Elgiloy, MP35N, Federstahl, rostfreiem Stahl, Titanium, Metallegierung mit Formspeichervermögen.

7. Vorrichtung (36) nach Anspruch 1, worin die Faser der Fasermatrix (14) einen Durchmesser von etwa 8 bis 10 Mikron und die Fasermatrix (14) eine Größe der Poren (15) von etwa 100 Mikron hat.

8. Vorrichtung (36) nach Anspruch 1, worin die Faser der Fasermatrix (14) ausgewählt ist aus Polyurethan, Nylon, PEBAX, Silikon, einem flexiblen Polymeren, das für Elektroaufspinnen geeignet ist, Polymilchsäure.

9. Vorrichtung (36) nach Anspruch 1, worin die Drähte (19) einen Durchmesser von etwa 0,0025 cm (0,001 Inch) bis 0,012 cm (0,005 Inch) haben.

10. Vorrichtung (36) nach Anspruch 1, ferner umfassend einen Führungsdraht (16), an dem der Filter (10) an einem distalen Ende des Führungsdrahtes (16) befestigt ist.

11. Vorrichtung (36) nach Anspruch 10, worin der Filter (10) eine zusammengefaltete Konfiguration und eine aufgespannte Konfiguration (40) hat, wobei
der Filter (10) in der zusammengefalteten Konfiguration zu dem Führungsdraht (16) hin zusammengefaltet ist; und
der Filter (10) in der aufgespannten Konfiguration (40) von dem Führungsdraht (16) nach außen aufgespannt ist.

12. Vorrichtung (36) nach Anspruch 1, worin die Filtermatrix (14) in einem regelmäßigen Muster gewoben ist.

13. Vorrichtung (36) nach Anspruch 1, worin die Filtermatrix (14) in einem Zufallsmuster gewoben ist.

14. Vorrichtung (36) nach Anspruch 1, worin die Fasermatrix (14) an einem distalen Ende des Rahmens, der proximalen Seite, durch ihn hindurch verwoben oder eine beliebige Kombination des vorstehenden ist.

15. Verfahren zur Herstellung einer medizinischen Vorrichtung (36) nach Anspruch 1 zum Ausfiltern von teilchenförmigem Material (28) aus einem Strömungsmittel in einem Lumen (22) des Körpers eines Patienten, indem ein Filter (10) mit einem Drahtrahmen (12) und einer Fasermatrix (14) gebildet wird, **gekennzeichnet durch** direktes Auspinnen auf elektrischem Wege von einer Faser auf den Faserrahmen (12).

16. Verfahren nach Anspruch 15, worin die Faser in einem fließfähigen Zustand aufgebracht wird.

17. Verfahren nach Anspruch 15, worin die Fasermatrix (14) in einem regelmäßigen oder unregelmäßigen Muster gewoben wird.

## Revendications

1. Dispositif médical (36) pour prévenir le passage du matériau de microparticules (28) entraîné dans un fluide s'écoulant par un lumen (22) comprenant:
- un filtre en état de collapsus et dilatable (10), où le filtre (10) présente un cadre de fils (12) comprenant une pluralité de fils (19) orientés pour définir un périmètre; et
- une matrice de fibres (14), incluant une multiplicité de fibres s'étendant sur un espacement ouvert défini par le périmètre pour définir une multiplicité de pores (15), fixée au cadre de fils (12), **caractérisé par** ce que
- la matrice de fibres (14) comprend un filage électrique de fibres directement sur le cadre de fil (12).

2. Dispositif (36) de la revendication 1, où le filtre (10) présente des pores (15) d'environ 100 microns et un pourcentage de zone ouverte d'environ 80%.

3. Dispositif (36) de la revendication 1, où les fils (19) ont un premier diamètre et la fibre de la matrice de fibres (14) a un diamètre plus petit.

4. Dispositif (36) de la revendication 1, où les fils (19) sont sélectionnés d'un métal ou polymère.

5. Dispositif (36) de la revendication 4, où les polymères sont sélectionnées d'entre nylons, téflon, tefzel, polyuréthanes, polymères à mémoire de forme.

6. Dispositif (36) de la revendication 4, où les métaux sont sélectionnés d'entre elgiloy, MP 35 N, acier à ressorts, acier inoxydable, titane, alliage de métal à mémoire de forme.

7. Dispositif (36) de la revendication 1, où la fibre de la matrice de fibres (14) présente un diamètre d'environ 8 à 10 microns et la matrice de fibres (14) présente une dimension de pore (15) d'environ 100 microns.

8. Dispositif (36) de la revendication 1, où la fibre de la matrice de fibres (14) est sélectionnée d'entre polyuréthane, nylon, PEBAX, silicone, un polymère flexible convenable pour filage électrique.

9. Dispositif (36) de la revendication 1, où les fils (19) ont un diamètre d'environ 0,0025 cm (0,001 pouces) à 0,012 cm (0,005 pouces).

10. Dispositif (36) de la revendication 1, comprenant de plus un fil-guide (16) ayant le filtre (10) attaché à une extrémité distale du fil-guide (16).

11. Dispositif (36) de la revendication 10, où le filtre (10) présente une configuration de collapsus et une configuration dilatable (40), où:
- le filtre (10) dans la configuration de collapsus est effondré vers le fil-guide (16); et
- le filtre (10) dans la configuration dilatable (40) est dilaté en dehors du fil-guide (16).

12. Dispositif (36) de la revendication 1, où la matrice de fibres (14) est tressée dans un modèle régulier.

13. Dispositif (36) de la revendication 1, où la matrice de fibres (14) est tressée dans un modèle irrégulier.

14. Dispositif (36) de la revendication 1, où la matrice de fibres (14) est sur un côté distal du cadre, le côté proximal est tressé par-là, ou n'importe laquelle combinaison de ci-dessus.

15. Procédé de faire un dispositif médical (36) selon la revendication 1, pour filtrer les microparticules (28) d'un fluide dans un lumen (22) d'un corps du patient, en formant un filtre (10) avec un cadre de fils (12) et une matrice de fibres (14), **caractérisé par** filage électrique d'une fibre directement sur le cadre de fils (12).

16. Procédé de la revendication 15, où la fibre est appliquée dans un état fluide.

17. Procédé de la revendication 15, où la matrice de fibres (14) est tressée dans un modèle régulier ou irrégulier.
